# EUROPEAN PATENT SPECIFICATION

(11) **EP 2 713 960 B1**
(45) Date of publication and mention of the grant of the patent: **22.03.2017**
(21) Application number: 12792704.4
(22) Date of filing: 01.06.2012
(51) Int. Cl.: A61F 2/64, A61F 2/38, A61F 2/30

(54) **PATIENT SPECIFIC INSTRUMENT**
PATIENTENSPEZIFISCHES INSTRUMENT
INSTRUMENT SPÉCIFIQUE À UN PATIENT

(30) Priority: 01.06.2011 US 201161491917 P
(43) Date of publication of application: 09.04.2014
(73) Proprietor: Smith & Nephew, Inc., Memphis, TN 38116 (US)
(72) Inventor: GIBSON, Luke Andrew, Southaven, Mississippi 38672 (US)
(74) Representative: Smith & Nephew
(86) International application number: PCT/US2012/040373
(87) International publication number: WO 2012/167016

(56) References cited:
- WO-A1-94/00056
- WO-A1-96/25114
- WO-A1-2009/001083
- GB-A- 2 447 702
- US-A- 5 709 689
- US-A1- 2004 153 087
- US-A1- 2004 260 301
- US-A1- 2008 243 127
- US-A1- 2009 088 753

## Description

### RELATED FIELDS

Patient specific instruments for use in, for instance, knee arthroplasty procedures such as total or partial knee replacements.

### BACKGROUND

Knee arthritis and trauma in various forms can cause loss of joint cartilage, including for example, osteoarthritis, excessive wear or sudden trauma, rheumatoid arthritis, or infectious arthritis. When joint cartilage is worn away, the bone beneath the cartilage is left exposed, and bone-on-bone contact can be very painful and damaging. Other types of problems can occur when the bone itself becomes diseased. Conventional solutions for these types of joint problems may be total or partial knee replacements.

In some knee arthroplasty procedures, for example, in preparing a femur to receive a femoral implant, a series of resections are made to the distal end of the femur. In "distal cut first" techniques, the first cut is a distal planar resection. Conventionally, after the distal resection has been made, a "four-in-one" cutting guide is positioned on the distal end of the femur relative to the planar distal resection. The four-in-one cutting guide typically includes four slots for guiding a cutting instrument to create a series of cuts on the distal femur. In some instances, the four-in-one cutting guide is placed on the distal femoral resection, making posterior bone cuts to the medial and lateral condyles, making posterior chamfer bone cuts to the medial and lateral condyles, making an anterior bone cut to the distal femur, and making an anterior chamfer bone cut.

It is important that the four-in-one cutting guide be positioned correctly on the distal end of the femur with respect to the distal cut since the position and orientation of the four-in-one guide (and the cuts made using it) will control the position and orientation of the femoral implant in at least some degrees of freedom. In some instances, properly positioning and orienting these resections to achieve optimal positions and orientations for the femoral implant can be difficult and time consuming. For example, some traditional four-in-one cutting guides have a planar surface that mates directly against the distal femur cut, and thus, in at least some instances, constitutes a planar joint that can be translated in at least two degrees of freedom and rotated in at least one degree of freedom (e. g., translated in medial/lateral and anterior/posterior directions and rotated in internal/external manners). The rotational and translational alignment of the guide may be set by drilling two pin holes into the distal femur. The four-in-one guide has two integrated pins that are then positioned inside the two drilled holes and the guide is then impacted against the distal cut. As force is applied to the center of the four-in-one cutting guide to secure the guide to the bone, a moment can be created that rotates the four-in-one cutting guide so that the position of the guide when it is secured to the bone is different from the intended position of the guide. Other factors can also cause improper position and/or orientation of the cutting guide on the distal cut. If the cutting guide is not properly positioned and oriented on the bone, the four cuts will be inaccurate and the femoral implant will not be implanted correctly, which may result in improper kinematics, biomechanics, or other undesirable performance.

Document GB 2 447 702 A discloses a femoral cutting guide comprising a plurality of guide slots for guiding the movement of cutting tools relative to a distal portion of a femur.

### SUMMARY

Instrumentation and systems including patient-matched instrumentation, and systems for facilitating orthopaedic procedures including knee arthroplasty procedures are disclosed herein. Certain implementations constitute alignment guides for aligning instrumentation on a distal femur. Portions of the guide may have surfaces that are generally a negative of the patient's anatomy, or are designed and adapted to substantially conform to a portion of the patient's anatomy, such as portions of the distal femur. Such surfaces can be formed using data obtained from the specific patient by conventional imaging devices or other techniques, such as x-ray, CT scans, MRI scans, or ultrasound, and using computer aided-design and manufacturing techniques.

In some implementations, the alignment guide includes one or more patient-matched surfaces for contacting un-altered anatomy of a patient as well as one or more planar surfaces (which may or may not also be customized to the specific patient) for contacting altered anatomy (e. g., a planar resection of the distal femur) of the patient.

In one general aspect, a femoral cutting guide includes guide slots for guiding the movement of cutting tools relative to a distal portion of a femur, a surface feature adapted to at least partially conform to a resected surface of the distal portion of the femur, and either a posterior-facing surface adapted to conform to an anterior, unaltered surface of the distal portion of the femur or an anterior-facing surface adapted to conform to a posterior, unaltered surface of the distal portion of the femur.

Implementations may optionally include one or more of the following features. For example, the guide slots may include an anterior slot for guiding an anterior resection of the distal portion of the femur and a posterior slot for guiding posterior resections on one of a medial or lateral condyle of the distal portion of the femur. The guide slots may further include an anterior chamfer slot for guiding an anterior chamfer resection of the distal portion of the femur and a posterior chamfer slot for guiding a posterior chamfer resection of the distal portion of the femur. The guide slots may include at least two posterior slots for guiding posterior resections of the medial and lateral condyle of the distal portion of the femur. The surface feature may include a substantially planar face adapted to, at least partially, conform to the resected surface of the distal portion of the femur. The cutting guide includes a posterior-facing surface adapted to conform to an anterior, unaltered surface of the distal portion of the femur and an anterior-facing surface adapted to conform to a posterior, unaltered surface of the distal portion of the femur. The anterior, unaltered surface of the distal portion of the femur includes anterior sulcus and patello-femoral groove portions of the distal portion of the femur. The posterior, unaltered surface of the distal portion of the femur includes a posterior sulcus portion of the distal portion of the femur. The anterior and posterior facing surfaces extend generally superiorly from the surface feature, and the surface feature extends between the anterior and posterior facing surfaces. The the posterior-facing surface and the anterior-facing surface are established in pre-surgical planning based on imaging data of the distal portion of the femur. The cutting guide may further include one or more apertures configured to guide the placement of provisional fixation pins for coupling the guide to the distal portion of the femur.

In another general aspect, a patient-specific femoral cutting guide includes an anterior portion, a posterior portion, and a substantially planar surface feature. The anterior portion including one or more surfaces adapted to substantially conform to an unaltered, anterior portion of a distal portion of a femur and at least one guide slot for guiding the movement of a cutting tool relative to the unaltered, anterior portion of the distal portion of the femur. The posterior portion including one or more surfaces adapted to substantially conform to an unaltered, posterior portion of the distal portion of the femur and at least one guide slot for guiding the movement of a cutting tool relative to the unaltered, posterior portion of the distal portion of the femur. The substantially planar surface feature extending between the anterior portion and the posterior portion and adapted to at least partially conform to a resected surface of the distal portion of the femur.

Implementations can optionally include one or more of the following features. For example, the relative position of the anterior portion relative to the posterior portion is adjustable. The relative position of the anterior portion relative to the posterior portion is adjustable in a manner that is substantially parallel to the resected surface of the distal portion of the femur when the cutting guide is positioned on the distal portion of the femur.

Also described herein is a method of resectioning a distal femur includes positioning a surface feature of a cutting guide against a first resected surface of the distal femur, positioning a posterior-facing surface of the cutting guide against an anterior, unaltered surface of the distal femur, positioning an anterior-facing surface of the cutting guide against a posterior, unaltered surface of the distal femur, and using the cutting guide to guide one or more cutting tools relative to the distal femur to form one or more resections of the distal femur.

Implementations can optionally include one or more of the following features. For example, using the cutting guide to form one or more resections of the distal femur includes using an anterior slot for guiding an anterior resection of the distal femur, using posterior slots for guiding posterior resections on the medial and lateral condyles of the distal femur, using anterior chamfer slots for guiding anterior chamfer resections of the distal femur, and using posterior chamfer slots for guiding posterior chamfer resections. The method may include using one or more fixation pins to secure the cutting guide in a particular location relative to the distal femur. Positioning the surface feature of the cutting guide against the first resected surface of the distal femur establishes a superior-inferior positioning of the cutting guide and varus-valgus orientation and angulation when viewed in a sagittal plane of the guide. Positioning the posterior-facing surface of the cutting guide against the anterior, unaltered surface of the distal femur and positioning the anterior-facing surface of the cutting guide against the posterior, unaltered surface of the distal femur establishes the anterior-posterior and medial-lateral positioning of the guide relative to the distal femur and the internal and external rotation of the guide relative to the distal femur. The surface feature includes a substantially planar face adapted to at least partially conform to the first resected surface of the distal femur.

### BRIEF DESCRIPTION OF THE DRAWINGS

Figure 1-3 are various views of an unresected, distal femur.
Figures 4-6 are various views of a distal cutting guide positioned on the distal femur of Figures 1-3.
Figures 7-9 are various views of the distal femur of Figures 1-3 after resection.
Figure 10 is a top plan view of an exemplary implementation of a patient-matched cutting guide.
Figure 11 is a left plan view of the cutting guide of Figure 10.
Figure 12 is a top perspective view of the cutting guide of Figure 10.
Figure 13 is a bottom plan view of the cutting guide of Figure 10.
Figure 14 is a lateral view of the cutting guide of Figure 10 positioned on the resected distal femur of Figures 7-9.
Figure 15 is a posterior plan view of the cutting guide of Figure 10 positioned on the resected distal femur of Figures 7-9.
Figure 16 is a distal view of the cutting guide of Figure 10 positioned on the resected distal femur of Figures 7-9.
Figure 17 is an anterior view of the cutting guide of Figure 10 positioned on the resected distal femur of Figures 7-9.
Figures 18-21 are various views of the distal femur of Figure 1 after resections have been made to various portions of the distal femur.
Figure 22 is a side view of an implant positioned on the resected distal femur.
Figure 23 is a side view of the implant of Figure 22.
Figure 24 is another implementation of a patient-matched cutting guide.
Figure 25 is a side view of the patient-matched cutting guide of Figure 24.
Figures 26-27 are schematics of an exemplary method and system for planning for and executing an orthopaedic procedure.

### DETAILED DESCRIPTION OF THE DRAWINGS

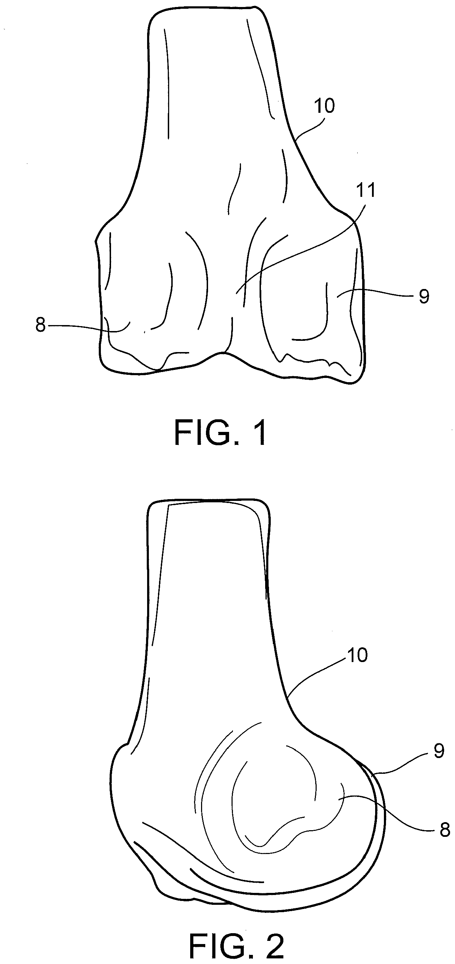

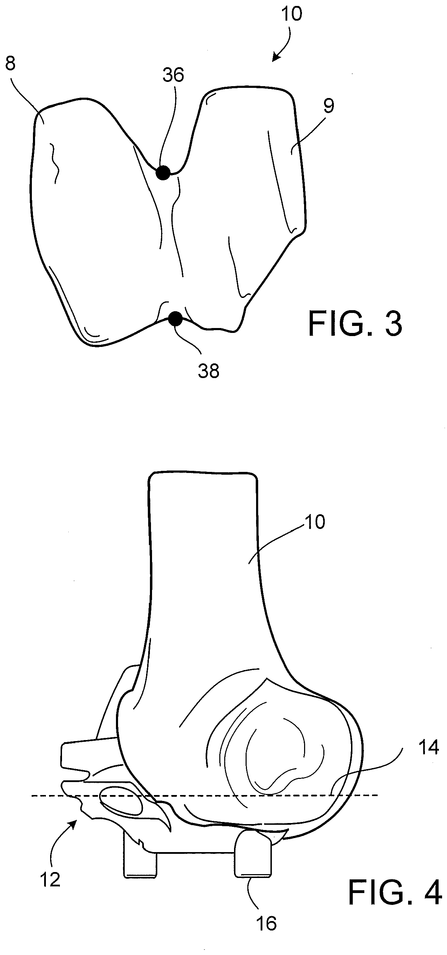

Figures 1-3 show a distal femur 10 before resection. Distal femur 10 includes a lateral condyle 8 and a medial condyle 9 and a patello-femoral groove 11 located between the lateral condyle 8 and the medial condyle 9, as shown in Figure 1. Distal femur 10 also includes an anterior sulcus 38 and a posterior sulcus 36, as shown in Figure 3.

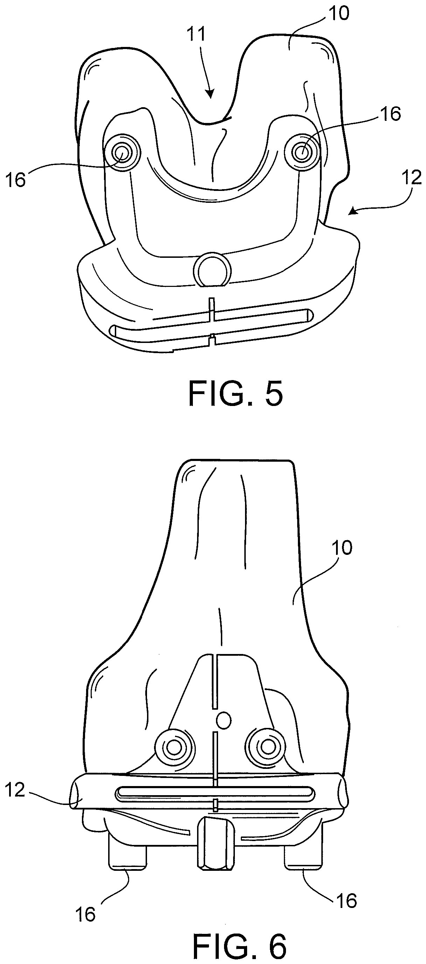

In some implementations, a distal femoral resection may be performed using conventional or non-conventional techniques and apparatus. For example, as shown in Figures 4-6, a patient-matched distal femoral cutting guide 12 may be navigated relative to the distal femur 10 and (optionally) pinned to the distal femur 10 to guide a reciprocating or oscillating saw or other cutting device to make the distal femoral planar resection along cut line 14. In some implementations, the cutting guide 12 includes pin holes 16 for drilling appropriate pin holes in the distal femur 10 to set implant rotation and/or for facilitating attachment of a different guide, such as a four-in-one cutting guide. In other implementations, a non-patient-matched cutting guide may be used to guide the distal femoral planar resection. In still other implementations, a patient-matched or non-patient-matched cutting guide may be used to make other resections initially before a distal resection (e. g., anterior-cut first techniques).

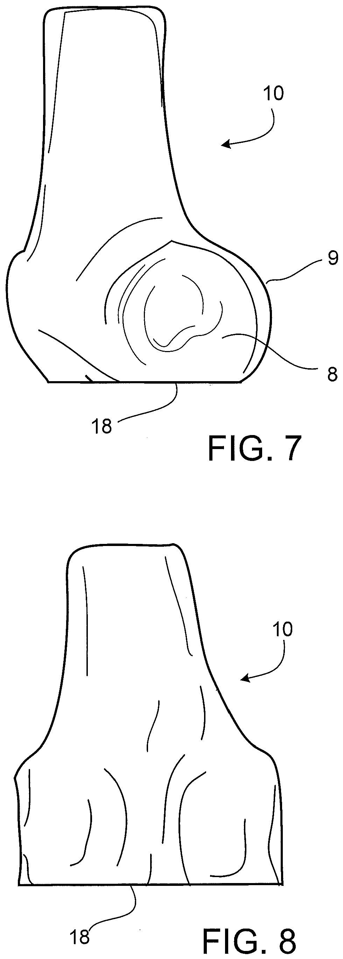

Figures 7-9 show the distal femur 10 after distal resection to define a resected surface 18 on the distal femur 10. In the particular implementation shown, resected surface 18 is substantially planar and extends substantially in a transverse plane relative to the distal femur 10 (e. g., as opposed to sagittal or coronal planes). As shown in Figures 7-9, distal portions of the medial condyle 9 and lateral condyle 8 are removed by the distal resection, while other portions of the distal femur 10 (e. g., anterior and posterior sulcus portions 38 and 36) are preserved and remain unaltered, at least at this stage of this implementation.

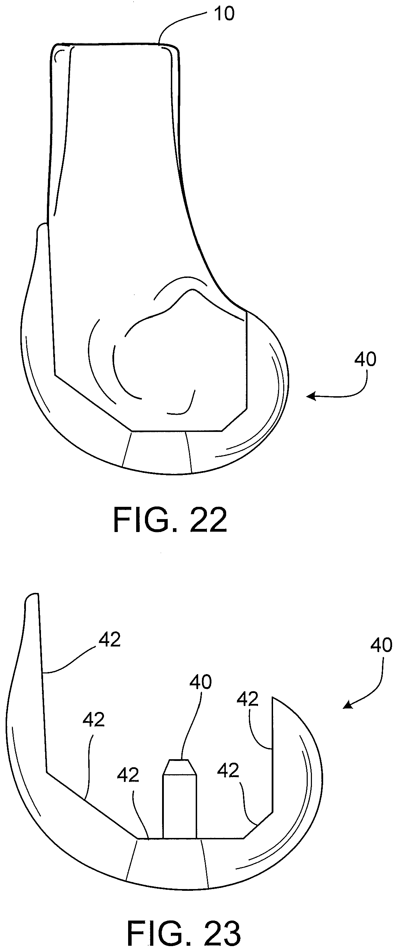

In the particular implementation shown, after the distal resection 18 has been made, a four-in-one cutting guide may be positioned on the distal femoral resection 18 to guide additional resections of the distal femur 10, such as one or more of a posterior resection, an anterior resection, and anterior and posterior chamfer resections. Such resections may prepare the distal femur to receive a femoral implant such as the implant 40 shown in Figure 23, which has bone-engaging surfaces 42 matching the distal, anterior 25, posterior 21 and chamfer resections 19, 23 created on the distal femur 10.

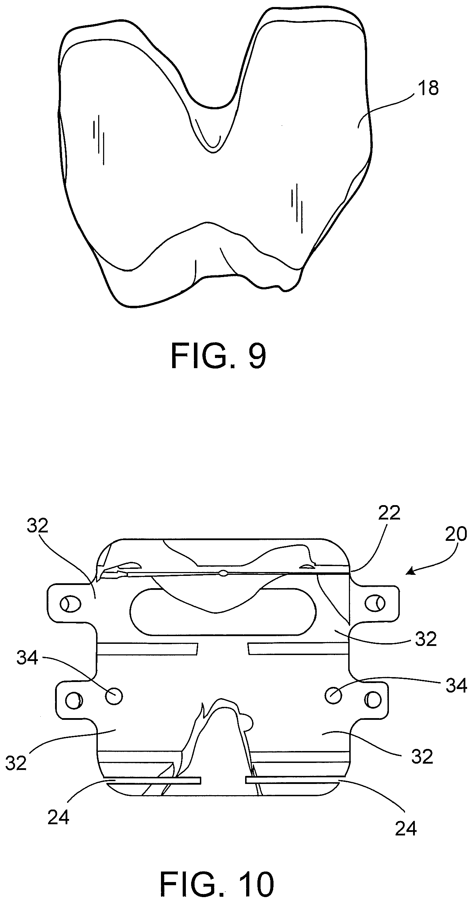

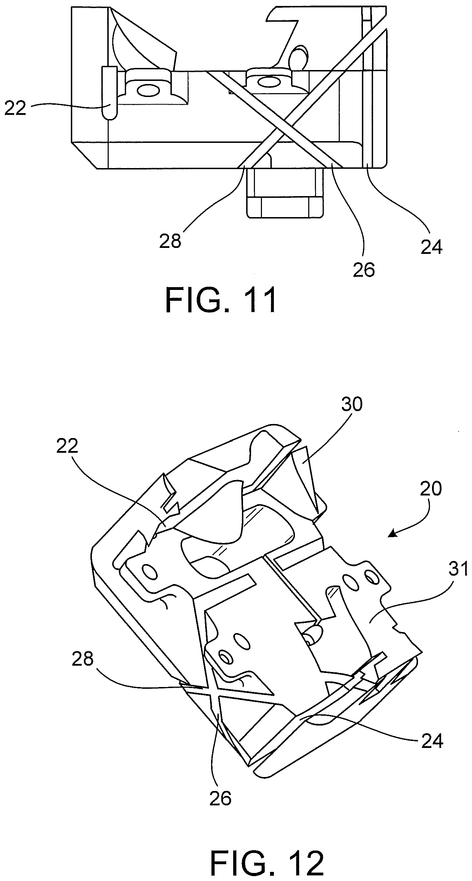

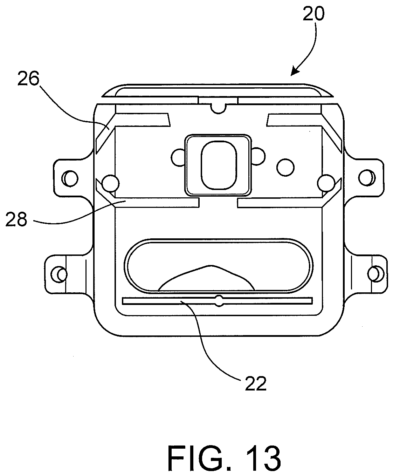

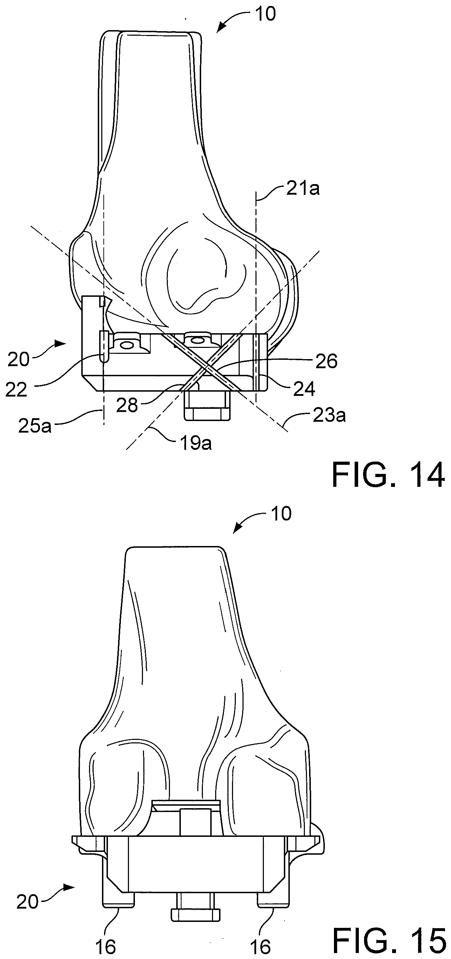

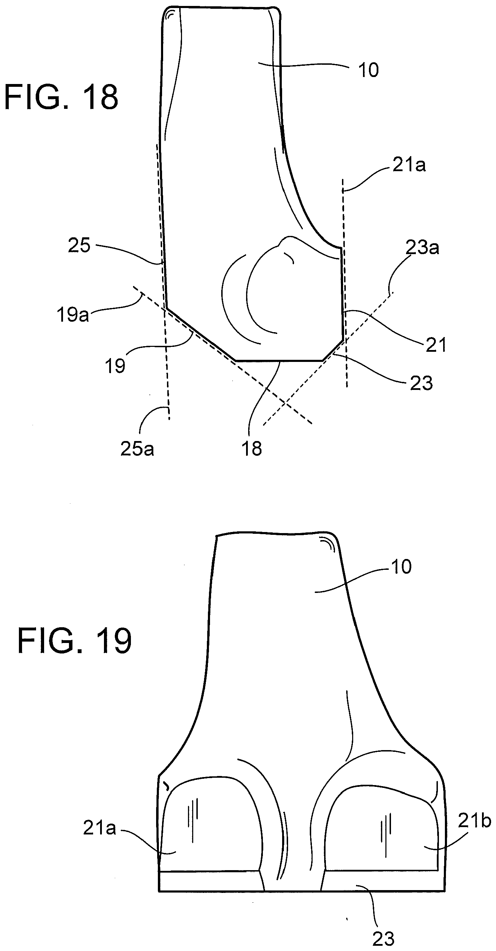

Figures 10-13 show an implementation of a patient-matched four-in-one cutting guide 20. Patient-matched cutting guide 20 includes a plurality of guide slots 22, 24, 26, and 28 and/or other guide surfaces for guiding the movement of cutting tools, such as reciprocating and oscillating saw blades, with respect to the distal femur 10. For example, referring also to Figs. 14 and 18, cutting guide 20 includes an anterior guide slot 22 for guiding an anterior resection 25 along a line 25a, posterior guide slots 24 for guiding posterior resections 21 along a line 21a on the medial and/or lateral condyles, anterior chamfer guide slots 26 (Fig. 11) for guiding anterior chamfer resections 23 along a line 23a, and posterior chamfer guide slots 28 (Fig. 11) for guiding posterior chamfer resections 19 along a line 19a. In some implementations, cutting guide 20 may include multiples of each guide slot to accommodate different size options for different sizes of implants. The cutting guide 20 shown also includes pin receiving holes 34 to facilitate securing the guide 20 to the patient's anatomy via, for example, provisional fixation pins.

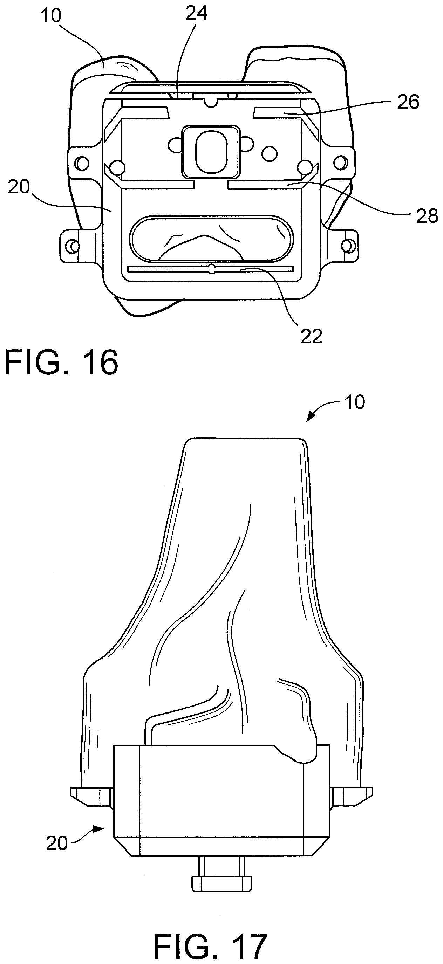

Figures 14-17 show various views of the patient-matched cutting guide 20 positioned and oriented on distal femur 10. The cutting guide 20 of Figure 10, when properly positioned and oriented on the patient's distal femur, establishes the positions and orientations of the four resections mentioned above.

In the particular implementation shown, the cutting guide 20 is configured to interact with both un-altered and altered anatomy on the distal femur 10 to determine the position and orientation of the cutting guide 20 with respect to the distal femur 10.

For instance, the cutting guide 20 includes a planar surface feature 32 that mates against and/or substantially conforms to the resected surface 18 of the femur 10. Positioning the planar surface feature 32 of the guide 20 against the resected surface 18 establishes the position and orientation of the guide 20 with respect to the femur 10 in certain degrees of freedom. For example, in this particular implementation, the mating of the planar surface feature 32 with the resected surface 18 establishes the superior-inferior positioning of the guide 20, as well as varus-valgus orientation and angulation when viewed in a sagittal plane of the guide 20.

The cutting guide 20 also includes two surfaces 30 and 31 that are adapted to at least substantially conform to the unique, un-altered geometry of the particular patient's anatomy (although in other implementations other numbers of such surfaces could be included). For instance, as shown in Figures 10-13, the cutting guide 20 includes a posterior facing surface 30 adapted to conform to anterior, un-altered anatomy of the patient's distal femur 10. In this particular implementation, the anterior un-altered anatomy includes anterior sulcus 38 and patello-femoral groove 11 portions of the distal femur 10. The cutting guide 20 shown in Figures 10-13 also includes an anterior-facing surface 31 adapted to conform to posterior, un-altered anatomy of the patient's distal femur 10, including posterior sulcus portion 36 of the distal femur 10. In the particular implementation shown, positioning the anterior and posterior-facing surfaces 30, 31 to conform to the anterior and posterior portions of the anatomy establishes the position and orientation of the guide 20 in additional degrees of freedom. In the specific implementation shown, the anterior and posterior-facing surfaces 30, 31 establish the position and orientation of the guide 20 in degrees of freedom other than those established by the interaction of the planar surface feature 32 with the planar resection 18 on the distal femur 10. Particularly, in this implementation, the anterior and posterior facing surfaces 30, 31 establish the anterior-posterior and medial-lateral positioning of the guide 20 relative to the distal femur 10, as well as the internal/external rotation of the guide 20 relative to the distal femur 10.

In the particular implementation shown, the anterior and posterior facing surfaces 30, 31 extend generally superiorly from the planar surface feature 32, which extends between those surfaces 30, 31. Additionally, as shown, unlike planar surface feature 32, the anterior and posterior-facing surfaces 30, 31 are, at least in this particular implementation, non-planar and undulate to correspond to the undulating anatomy they are designed to interact with and conform to.

In other implementations, cutting guides do not necessarily need to include both anterior-facing patient-matched surface 31 and posterior-facing patient-matched surface 30, and may include only one of such surfaces. In other implementations, cutting guides may include more than these two surfaces that are also configured to conform to the patient's un-altered anatomy.

In some implementations, instead of integrated pins, as were included in some conventional four-in-one cutting guides, the patient-matched cutting guide 20 includes at least two apertures 34 for receiving fixation pins to secure the guide to the distal femur 10. The patient-matched surfaces 30, 31 position the cutting guide 20 relative to the resected surface 18 in only one way against the anatomy before the guide is secured. Instead of using two integrated pins, the apertures 34 should be aligned with the holes that were drilled in the distal femur using cutting guide 12. The apertures 34 can provide a check that the cutting guide 20 is properly aligned and that all five cuts will be in alignment per the surgical plan. If the apertures 34 are not aligned with the holes created by the distal cutting block, it could indicate that the distal cutting block or the 4-in-1 cutting guide 20 was not secured in the proper location. Other holes can also be used to secure the cutting guide 20 to the bone. As compared with conventional cutting guides using integrated pins, the cutting guide 20 is properly positioned both before and after it is secured. As described above, with conventional four-in-one cutting guides, a force directed toward the center of the cutting guide 20 to secure the guide 20 to the bone 10 often created a moment that rotated the cutting guide 20 during impaction so that the final position of the guide 20 was different than planned position of the guide 20, which caused inaccurate cuts.

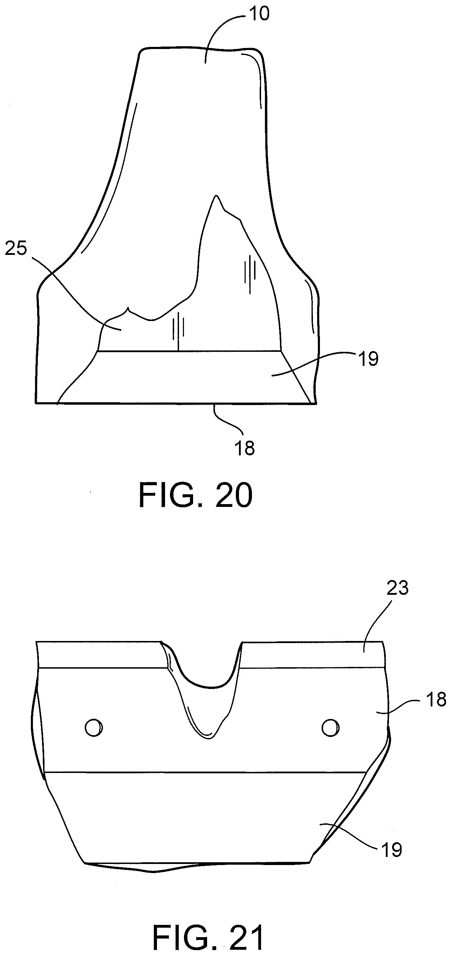

Figures 18-21 show the distal femur 10 of Figure 1 after the various resections using the patient-matched cutting guide of Figure 10. As shown in Figures 18-21, the anterior 25 and posterior 21 resections are substantially parallel, and the distal resection 18 is substantially perpendicular to the anterior and posterior resections 25, 21. Other resection layouts, positions and orientations of resections are also possible and within the scope of the present disclosure. Following resection of the distal femur 10, an implant, such as the implant 40 shown in Fig. 22 may be positioned and secured to the resected distal femur 10. The implant 40 includes a number of inner surfaces 42 (Fig. 23) that substantially mate with and/or align with the resected surfaces 18, 19, 21, 23, 25 to help position the implant 40 on the distal femur 10.

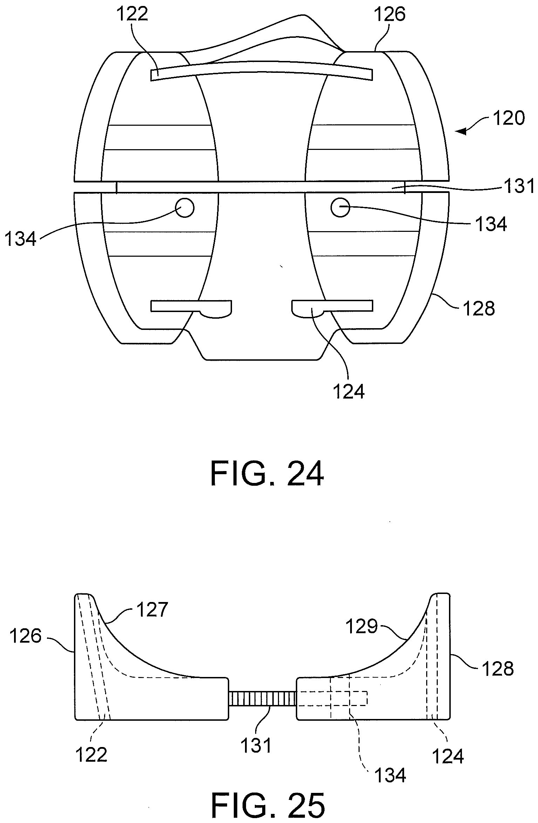

Figs. 24-25 illustrate another cutting guide 120 according to an alternate implementation. Cutting guide 120 includes an anterior portion 126 and a posterior portion 128, which can be two separate pieces. Anterior portion 126 and posterior portion 128 each include one or more patient matched surfaces 127, 129. The anterior portion 126 also includes an anterior guide slot 122 for making an anterior cut 25 and the posterior portion 128 includes a posterior guide slot 124 for making a posterior cut 21. Because the two portions 126 and 128 may be separate pieces, they may be separated / adjusted relative to one another in a manner that is parallel to the distal resected surface 18. The relative position of each portion to the other can be adjusted in increments that correlate with various sized implants, which allows a surgeon to either position the cutting guide 120 against the posterior condyles and adjust the size and/or position by posterior referencing, or position the cutting guide 120 against the anterior sulcus 38 and adjust the size and/or position by anterior referencing. In this way, the surgeon can switch between posterior and anterior referencing intra-operatively, as well as up-size or downsize the implant intra-operatively. A connecting portion 131 connects anterior portion 126 and posterior portion 128 and allows for adjustment of position of the anterior portion 126 relative to the posterior portion 128. Connecting portion 131 may have hash marks or other indices to indicate the amount of additional bone to be removed by separating anterior portion 126 and posterior portion 128. Anterior portion 126 and posterior portion 128 may also have fixation openings or pin holes 134 to facilitate securing the guide 120 to the patient's anatomy via, for example, provisional fixation pins.

Also described herein is a method of creating a patient-matched cutting guide. Imaging data taken by any known means such as x-ray, CT scans, MRI scans, ultrasound, etc. is used to obtain patient-specific data about the femur to be resected and other anatomy of potential relevance, such as imaging data of the femoral head and ankle to allow determination of a mechanical axis of the leg. In one implementation, the patient-specific data is used to build a model of the patient's anatomy. Then, the model is used to plan the distal resection. In some implementations, a patient-matched guide is built to guide the distal resection. Next, using the patient-specific data, as well as data about the planned distal resection (including orientation and position of that resection), cutting guide 20 or 120 may be created to conform to the un-altered anatomy of the patient, as well as the altered (resected) anatomy so that the planar face of the cutting guide fits properly on the resected surface after the distal resection has been made. In particular, the imaging data is used to create patient-matched surfaces 30 and/or 31 that will mate against the uncut bone to help guide the remaining resections and to create planar surface 32 that will mate against the distal resection. For example, in some implementations, cutting guide 12 (shown in Figs. 4-6), which is used to make the distal resection, is created using imaging data of the patient's specific anatomy and then the bone to be resected during the distal resection is subtracted from the image and the cutting guide 20 is created from such image.

According to another non-limiting method, a patient may contact a health care provider regarding joint pain, such as knee pain. The health care provider may then refer the patient to an imaging facility. A technician images the joint, such as by MRI, CT, X-ray, or ultrasound. In one implementation, the technician transmits the image data to a cutting block manufacturer and/or to the health care provider. The cutting block manufacturer manufactures the distal resection block and the four-in-one block using the image data. In some implementations, the cutting block manufacturer or its representative are located at a surgical site facility. For instance, the distal resection block and/or the four-in-one cutting block may be manufactured at or near the site facility where the joint replacement takes place. The health care provider and the cutting block manufacturer may communicate regarding the requirements of at least one of the cutting blocks. Communication may take place via a representative or agent, a telephone network, a computer network, or by any other suitable mode. In one implementation, the patient then visits a surgical site facility for joint replacement, and the health care provider confirms the cutting blocks are matched to the patient. The health care provider places the distal cutting block on the patient. The health care provider makes a distal cut. The health care provider places the patient matched four-in-one cutting block on the patient and makes additional cuts. The health care provider implants a joint prosthesis, such as a knee prosthesis.

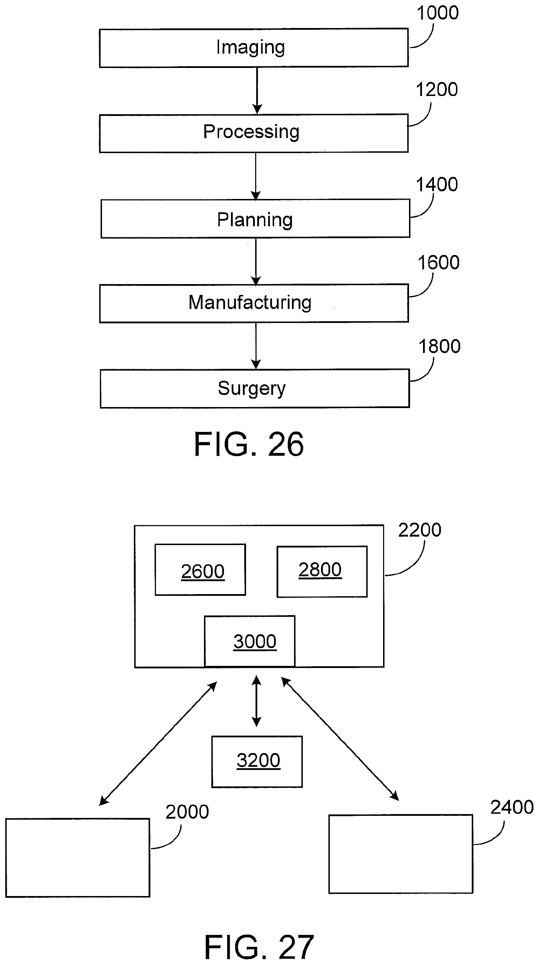

One non-limiting example of a method of planning for and executing an orthopaedic procedure is shown in Figure 26, which, in this particular implementation, is a knee arthroplasty procedure. As schematically shown by Figure 26, the general steps may include imaging 1000, processing 1200, planning 1400, manufacturing 1600 and performing the surgery 1800, although, in other implementations, at least some of these steps may be optional and other steps could be included in addition to those shown in Figure 26.

A wide variety of systems may be utilized in performing the procedure shown in Figure 26. Figure 27 schematically shows one implementation of such a system for facilitating the steps of imaging, processing, planning and manufacturing. Figure 27 schematically illustrates imaging 2000, computing 2200, and manufacturing devices 2400.

In some implementations, including the implementation shown in Figure 27, steps such as image processing 1200 and surgical planning 1400 steps may be carried out, wholly or at least partially, using a computing device 2200. The computing device 2200 may be part of or remote from the device or devices used to image 2000 the patient and the device or devices 2400 used to custom manufacture instrumentation, implants or other devices for carrying out the procedure, and may receive or access data reflecting the images obtained of the patient through any appropriate communication medium, including wireline, wireless, optical, magnetic, or solid state communication mediums. The computing device 2200 represented in Fig. 27 includes a processor 2600 that can execute code stored on a computer-readable medium, such as a memory 2800. The computing device 2200 may be any device that can process data and execute code that is a set of instructions to perform actions. Examples of the computing device 2200 include a database server, a web server, desktop personal computer, a laptop personal computer, a server device, a handheld computing device, a mobile device, or combinations thereof.

In some implementations, the processor 2600 may include a microprocessor, an application-specific integrated circuit (ASIC), a state machine, or other suitable processor. The processor 2600 may include one processor or any number of processors, and may access code stored in the memory 2800. The memory 2800 may be any non-transitory computer-readable medium capable of tangibly embodying code. The memory 2800 may include electronic, magnetic, or optical devices capable of providing processor 2600 with executable code. Examples of the memory 2800 include random access memory (RAM), read-only memory (ROM), a floppy disk, compact disc, digital video device, magnetic disk, an ASIC, a configured processor, or other storage device.

In some implementations, including the implementation shown in Figure 27, the computing device 200 may share and/or receive data with additional components through an input/output (I/O) interface 3000. The I/O interface 3000 may include a USB port, an Ethernet port, a serial bus interface, a parallel bus interface, a wireless connection interface, or any suitable interface capable of allowing data transfers between the computing device and another component. The additional components may include components such as an information database 3200. In other implementations, the computing device 2200 includes the information database 3200.

In the implementations illustrated by Figures 26 and 27, the patient's anatomy of interest may be imaged 1000 using one or more non-invasive imaging technologies, such as, but not limited to, computed tomography (CT), magnetic resonance imaging (MRI), x-ray, digital x-ray, ultrasound or other imaging technologies. In implementations that utilize imaging technologies such as CT, MRI or others, one or more sets of parallel image slices of the patient's anatomy may be obtained, such as, but not limited to, a series of transverse slices, sagittal slices, coronal slices, other angulations of slices, or combinations of series thereof. In some implementations, multiple imaging technologies may be used for the same patient (e. g., x-ray for broader imaging of the overall patient, including other joints, and MRI for the joint of particular interest). The images of the patient's anatomy may, optionally, also include images of existing implant(s) or portions thereof. In some implementations, non-image based technologies may be utilized to obtain patient specific information about the patient's anatomy and geometries or other features associated therewith.

Image processing 1200 is the next step in the implementation of Figs. 26 and 27, in which at least some of the images may be processed to create an accurate 3-D model, other multidimensional representation, or other virtual construct representing the geometries and/or selected features of the patient's particular anatomy. In some implementations, such processing involves segmentation of the images (such as separation of at least one set of image slices) to distinguish the anatomy and other structures of interest from the surrounding anatomy and other structures appearing in the image. For example, the images associated with the femoral condyles may be segmented.

In various implementations, segmentation may be accomplished by manual, automated, or semi-automated processes. For instance, in some implementations, a technician or other user may (with the assistance of computer assisted design hardware and/or software or other functionality) manually trace the boundary of the anatomy and other structures of interest in each image slice. Alternatively, in some implementations, algorithms or other automated or semi-automated processes could be used to automatically identify the boundaries of interest. In some implementations, only key points on the anatomy or other structures of interest need be segmented. Image processing 1200 such as described above may be used to make a three dimensional model of the patient's anatomy and other features of interest.

The 3D model or other construct representing the patient's anatomy may be used for pre-surgical planning 1400 of the knee arthroplasty procedure. In some implementations, pre-surgical planning 1400 can include one or more of identifying a desired position and orientation of the distal resection and/or the patient-matched surfaces 30 and 31. In various implementations, the planning may be carried out using manual, semi-automated or automated functionality.

The patient-matched guide 20 or 120 may include one or more surfaces that are specifically designed to mimic the patient's particular anatomy (or portions thereof) as determined, for instance, by the 3D model of the anatomy. For instance, in some implementations, the patient-matched surface or surfaces can be a negative mold of the patient's anatomy such that it uniquely conforms to the patient's anatomy in one particular position and orientation. In other words, the patient-matched surface or surfaces may facilitate achieving a desired position and/or orientation of the patient-matched guide 20 or 120 with respect to the patient's particular anatomy because the patient-matched surface will allow the patient-matched guide 20 or 120 to fully seat on the patient's particular anatomy only when the patient-matched guide 20 or 120 is in the desired position and/or orientation.

In some implementations, the geometries and other aspects of the patient-matched surface may be determined in the planning stage by applying a blank of the patient-matched guide 20 or 120 (e. g. a wire-frame or similar digital representation of a blank of the patient-matched instrument) to the 3-D model of the patient's anatomy such that the patient-matched guide 20 or 120 is in the desired position and orientation with respect to the patient's anatomy, and then removing from or adding to portions of the blank to create the patient-matched surface conforming to the surface of the patient's anatomy. In some implementations, earlier processes performed during the planning stage will determine, at least partially or wholly, the position and/or orientation of the blank relative to the 3-D model of the patient's anatomy. For instance, the planned resection position and orientation, in combination with the 3D model, could be used to define the particular shape and other attributes of the patient matched guide.

Once designed, the patient-matched guide 20 or 120 may be manufactured 1600 using any number of known technologies, including, but not limited to, selective laser sintering, 3D printing, stereo-lithography, or other rapid production or custom manufacturing technologies. In some implementations, the rapid production equipment can be remote from the systems involved in the planning and/or designing of the patient-matched instruments, and data or other information sufficient to manufacture the patient-matched instrument(s) can be exported from the planning / design systems to the manufacturing systems in any desirable format.

One of ordinary skill in the art will recognize that additions, deletions, substitutions or other modifications may be made to the non-limiting implementations described above without departing from the scope of the present invention.

## Claims

1. A femoral cutting guide (20, 120) comprising:
a plurality of guide slots (22, 24, 26, 28) for guiding the movement of cutting tools relative to a distal portion of a femur;
a surface feature (32) adapted to at least partially conform to a resected surface of the distal portion of the femur;
a posterior-facing surface (31) which is undulating to conform to an anterior, unaltered surface of the distal portion of the femur and an anterior-facing surface (30) which is undulating to conform to a posterior, unaltered surface of the distal portion of the femur, wherein the anterior, unaltered surface of the distal portion of the femur comprises anterior sulcus and patello-femoral groove portions of the distal portion of the femur and further wherein the posterior, unaltered surface of the distal portion of the femur comprises a posterior sulcus portion of the distal portion of the femur.

2. The cutting guide of claim 1, wherein the plurality of guide slots comprise an anterior slot (22) for guiding an anterior resection of the distal portion of the femur and a posterior slot (24) for guiding posterior resections on one of a medial or lateral condyle of the distal portion of the femur.

3. The cutting guide of claim 2, wherein the plurality of guide slots further comprise an anterior chamfer slot (26) for guiding an anterior chamfer resection of the distal portion of the femur and a posterior chamfer slot (28) for guiding a posterior chamfer resection of the distal portion of the femur.

4. The cutting guide of claim 2, wherein the plurality of guide slots comprise at least two posterior slots for guiding posterior resections of the medial and lateral condyle of the distal portion of the femur.

5. The cutting guide of claim 1, wherein the surface feature comprises a substantially planar face adapted to at least partially conform to the resected surface of the distal portion of the femur.

6. The cutting guide of claim 1, wherein the cutting guide comprises a posterior- facing surface adapted to conform to an anterior, unaltered surface of the distal portion of the femur and an anterior-facing surface adapted to conform to a posterior, unaltered surface of the distal portion of the femur.

7. The cutting guide of claim 1, wherein the anterior and the posterior surfaces establish the anterior-posterior and medial-lateral positioning of the guide as well as internal/ external rotation of the guide relative to the distal femur,

8. The cutting guide of claim 1, wherein the anterior and posterior facing surfaces extend generally superiorly from the surface feature, and wherein the surface feature extends between the anterior and posterior facing surfaces.

9. The cutting guide of claim 1, wherein the posterior-facing surface and the anterior-facing surface are established in pre-surgical planning based on imaging data of the distal portion of the femur.

10. The cutting guide of any one of the preceding claims, further comprising one or more apertures configured to guide the placement of provisional fixation pins for coupling the guide to the distal portion of the femur.

11. The cutting guide of claim 1, wherein:
the posterior-facing surface is comprised in an anterior portion which further comprises at least one guide slot for guiding the movement of a cutting tool relative to the unaltered, anterior portion of the distal portion of the femur;
the anterior-facing surface is provided on a posterior portion which further comprises at least one guide slot for guiding the movement of a cutting tool relative to the unaltered, posterior portion of the distal portion of the femur; and wherein the
surface feature is a substantially planar surface feature extending between the anterior portion and the posterior portion.

12. The cutting guide of claim 11, wherein the relative position of the anterior portion relative to the posterior portion is adjustable.

13. The cutting guide of claim 12, wherein the relative position of the anterior portion relative to the posterior portion is adjustable in a manner that is substantially parallel to the resected surface of the distal portion of the femur when the cutting guide is positioned on the distal portion of the femur.

14. The cutting guide of claim 11, wherein the anterior portion and the posterior portion are separate pieces.

## Patentansprüche

1. Eine Femurschneideführung (20, 120), beinhaltend:
eine Vielzahl von Führungsschlitzen (22, 24, 26, 28) zum Führen der Bewegung von Schneidewerkzeugen relativ zu einem distalen Abschnitt eines Femurs;
ein Oberflächenmerkmal (32), das angepasst ist, um mindestens teilweise mit einer resezierten Oberfläche des distalen Abschnitts des Femurs übereinzustimmen;
eine posterior weisende Oberfläche (31), die gewellt ist, um mit einer anterioren, nicht geänderten Oberfläche des distalen Abschnitts des Femurs übereinzustimmen, und eine anterior weisende Oberfläche (30), die gewellt ist, um mit einer posterioren, nicht geänderten Oberfläche des distalen Abschnitts des Femurs übereinzustimmen, wobei die anteriore, nicht geänderte Oberfläche des distalen Abschnitts des Femurs Anterior-Sulcus- und Patello-Femur-Rinnen-Abschnitte des distalen Abschnitts des Femurs beinhaltet und wobei ferner die posteriore, nicht geänderte Oberfläche des distalen Abschnitts des Femurs einen Posterior-Sulcus-Abschnitt des distalen Abschnitts des Femurs beinhaltet.

2. Schneideführung gemäß Anspruch 1, wobei die Vielzahl von Führungsschlitzen einen anterioren Schlitz (22) zum Führen einer anterioren Resektion des distalen Abschnitts des Femurs und einen posterioren Schlitz (24) zum Führen posteriorer Resektionen an einem von einem medialen oder lateralen Kondylus des distalen Abschnitts des Femurs beinhaltet.

3. Schneideführung gemäß Anspruch 2, wobei die Vielzahl von Führungsschlitzen ferner einen anterioren Schrägschlitz (26) zum Führen einer anterioren Schrägresektion des distalen Abschnitts des Femurs und einen posterioren Schrägschlitz (28) zum Führen einer posterioren Schrägresektion des distalen Abschnitts des Femurs beinhaltet.

4. Schneideführung gemäß Anspruch 2, wobei die Vielzahl von Führungsschlitzen mindestens zwei posteriore Schlitze zum Führen posteriorer Resektionen des medialen und lateralen Kondylus des distalen Abschnitts des Femurs beinhaltet.

5. Schneideführung gemäß Anspruch 1, wobei das Oberflächenmerkmal eine im Wesentlichen planare Fläche beinhaltet, die angepasst ist, um mindestens teilweise mit der resezierten Oberfläche des distalen Abschnitts des Femurs übereinzustimmen.

6. Schneideführung gemäß Anspruch 1, wobei die Schneideführung eine posterior weisende Oberfläche, die angepasst ist, um mit einer anterioren, nicht geänderten Oberfläche des distalen Abschnitts des Femurs übereinzustimmen, und eine anterior weisende Oberfläche, die angepasst ist, um mit einer posterioren, nicht geänderten Oberfläche des distalen Abschnitts des Femurs übereinzustimmen, beinhaltet.

7. Schneideführung gemäß Anspruch 1, wobei die anteriore und die posteriore Oberfläche die Anterior-posterior- und Medial-lateral-Positionierung der Führung sowie die interne/externe Drehung der Führung relativ zu dem distalen Femur festsetzen.

8. Schneideführung gemäß Anspruch 1, wobei sich die anterior und posterior weisenden Oberflächen im Allgemeinen superior von dem Oberflächenmerkmal erstrecken und wobei sich das Oberflächenmerkmal zwischen den anterior und posterior weisenden Oberflächen erstreckt.

9. Schneideführung gemäß Anspruch 1, wobei die posterior weisende Oberfläche und die anterior weisende Oberfläche bei einer prächirurgischen Planung auf Basis von Abbildungsdaten des distalen Abschnitts des Femurs festgesetzt werden.

10. Schneideführung gemäß einem der vorhergehenden Ansprüche, die ferner eine oder mehrere Öffnungen beinhaltet, welche konfiguriert sind, um die Platzierung von Stiften zur vorläufigen Fixierung zum Koppeln der Führung an den distalen Abschnitt des Femurs zu führen.

11. Verfahren gemäß Anspruch 1, wobei:
die posterior weisende Oberfläche in einem anterioren Abschnitt beinhaltet ist, welcher ferner mindestens einen Führungsschlitz zum Führen der Bewegung eines Schneidewerkzeugs relativ zu dem nicht geänderten, anterioren Abschnitt des distalen Abschnitts des Femurs beinhaltet;
die anterior weisende Oberfläche auf einem posterioren Abschnitt bereitgestellt ist, welcher ferner mindestens einen Führungsschlitz zum Führen der Bewegung eines Schneidewerkzeugs relativ zu dem nicht geänderten, posterioren Abschnitt des distalen Abschnitts des Femurs beinhaltet; und wobei
das Oberflächenmerkmal ein im Wesentlichen planares Oberflächenmerkmal ist, das sich zwischen dem anterioren Abschnitt und dem posterioren Abschnitt erstreckt.

12. Schneideführung gemäß Anspruch 11, wobei die relative Position des anterioren Abschnitts relativ zu dem posterioren Abschnitt einstellbar ist.

13. Schneideführung gemäß Anspruch 12, wobei die relative Position des anterioren Abschnitts relativ zu dem posterioren Abschnitt auf eine Art und Weise einstellbar ist, die zu der resezierten Oberfläche des distalen Abschnitts des Femurs im Wesentlichen parallel ist, wenn die Schneideführung auf dem distalen Abschnitt des Femurs positioniert ist.

14. Schneideführung gemäß Anspruch 11, wobei der anteriore Abschnitt und der posteriore Abschnitt separate Teile sind.

## Revendications

1. Un guide de coupe fémorale (20, 120) comprenant :
une pluralité de fentes de guidage (22, 24, 26, 28) pour guider le déplacement d'outils de coupe par rapport à une partie distale d'un fémur ;
une caractéristique de surface (32) conçue pour se conformer au moins partiellement à une surface réséquée de la partie distale du fémur ;
une surface frontale postérieure (31) qui ondule pour se conformer à une surface non altérée antérieure de la partie distale du fémur et une surface frontale antérieure (30) qui ondule pour se conformer à une surface non altérée postérieure de la partie distale du fémur, dans lequel la surface non altérée antérieure de la partie distale du fémur comprend des parties de rainure fémoro-patellaire et de sillon antérieur de la partie distale du fémur et dans lequel en outre la surface non altérée postérieure de la partie distale du fémur comprend une partie de sillon postérieur de la partie distale du fémur.

2. Le guide de coupe de la revendication 1, dans lequel la pluralité de fentes de guidage comprend une fente antérieure (22) pour guider une résection antérieure de la partie distale du fémur et une fente postérieure (24) pour guider des résections postérieures sur un condyle parmi un condyle médial ou latéral de la partie distale du fémur.

3. Le guide de coupe de la revendication 2, dans lequel la pluralité de fentes de guidage comprend en outre une fente de chanfrein antérieur (26) pour guider une résection de chanfrein antérieur de la partie distale du fémur et une fente de chanfrein postérieur (28) pour guider une résection de chanfrein postérieur de la partie distale du fémur.

4. Le guide de coupe de la revendication 2, dans lequel la pluralité de fentes de guidage comprend au moins deux fentes postérieures pour guider des résections postérieures du condyle médial et latéral de la partie distale du fémur.

5. Le guide de coupe de la revendication 1, dans lequel la caractéristique de surface comprend une face substantiellement planaire conçue pour se conformer au moins partiellement à la surface réséquée de la partie distale du fémur.

6. Le guide de coupe de la revendication 1, dans lequel le guide de coupe comprend une surface frontale postérieure conçue pour se conformer à une surface non altérée antérieure de la partie distale du fémur et une surface frontale antérieure conçue pour se conformer à une surface non altérée postérieure de la partie distale du fémur.

7. Le guide de coupe de la revendication 1, dans lequel les surfaces antérieure et postérieure établissent le positionnement antérieur-postérieur et médial-latéral du guide ainsi qu'une rotation interne/externe du guide par rapport au fémur distal.

8. Le guide de coupe de la revendication 1, dans lequel les surfaces frontales antérieure et postérieure s'étendent de façon généralement supérieure à partir de la caractéristique de surface, et dans lequel la caractéristique de surface s'étend entre les surfaces frontales antérieure et postérieure.

9. Le guide de coupe de la revendication 1, dans lequel la surface frontale postérieure et la surface frontale antérieure sont établies lors de la planification préopératoire sur la base de données d'imagerie de la partie distale du fémur.

10. Le guide de coupe de l'une quelconque des revendications précédentes, comprenant en outre une ou plusieurs ouvertures configurées pour guider la mise en place de broches de fixation provisoires afin de coupler le guide à la partie distale du fémur.

11. Le guide de coupe de la revendication 1, dans lequel :
la surface frontale postérieure est comprise dans une partie antérieure qui comprend en outre au moins une fente de guidage pour guider le déplacement d'un outil de coupe par rapport à la partie antérieure non altérée de la partie distale du fémur ;
la surface frontale antérieure est fournie sur une partie postérieure qui comprend en outre au moins une fente de guidage pour guider le déplacement d'un outil de coupe par rapport à la partie postérieure non altérée de la partie distale du fémur ; et dans lequel la caractéristique de surface est une caractéristique de surface substantiellement planaire s'étendant entre la partie antérieure et la partie postérieure.

12. Le guide de coupe de la revendication 11, dans lequel la position relative de la partie antérieure par rapport à la partie postérieure est réglable.

13. Le guide de coupe de la revendication 12, dans lequel la position relative de la partie antérieure par rapport à la partie postérieure est réglable d'une manière qui est substantiellement parallèle à la surface réséquée de la partie distale du fémur lorsque le guide de coupe est positionné sur la partie distale du fémur.

14. Le guide de coupe de la revendication 11, dans lequel la partie antérieure et la partie postérieure sont des pièces distinctes.
